(19) Europäisches Patentamt
European Patent Office
Office européen des brevets

(11) **EP 1 543 146 B1**

(12) **EUROPEAN PATENT SPECIFICATION**

(45) Date of publication and mention of the grant of the patent:
**21.12.2011 Bulletin 2011/51**

(51) Int Cl.:
***C12Q 1/04*** *(2006.01)*     ***G01N 21/33*** *(2006.01)*

(21) Application number: **03784997.3**

(22) Date of filing: **07.08.2003**

(86) International application number:
**PCT/US2003/024728**

(87) International publication number:
**WO 2004/015136 (19.02.2004 Gazette 2004/08)**

(54) **INTERPRETATION MODEL FOR THE UV-VIS SPECTRA OF MICROORGANISMS**

MODELL ZUR INTERPRETATION DER UV-VIS-SPEKTREN VON MIKROORGANISMEN

MODELE D'INTERPRETATION POUR LES SPECTRES UV-VIS DE MICROORGANISMES

(84) Designated Contracting States:
**AT BE BG CH CY CZ DE DK EE ES FI FR GB GR HU IE IT LI LU MC NL PT RO SE SI SK TR**

(30) Priority: **07.08.2002 US 214264**

(43) Date of publication of application:
**22.06.2005 Bulletin 2005/25**

(73) Proprietor: **UNIVERSITY OF SOUTH FLORIDA Tampa, FL 33620-7900 (US)**

(72) Inventors:
• **GARCIA-RUBIO, Luis, H.**
  **Temple Terrace, FL 33617 (US)**
• **ALUPOAEI, Catalina, Elena**
  **Tampa, FL 33613 (US)**

(74) Representative: **Chaillot, Geneviève et al**
**Cabinet Chaillot**
**16-20 Avenue de l'Agent Sarre**
**B.P. 74**
**92703 Colombes Cedex (FR)**

(56) References cited:
WO-A2-02/04947    GB-A- 2 335 981
US-A- 5 164 301    US-A- 5 616 457
US-A- 5 784 162    US-A1- 2001 024 800

• **MATTLEY Y D; GARCIA-RUBIO L H: "Multiwavelength spectroscopy for the detection, identification and quantification of cells" PROCEEDINGS OF SPIE - THE INTERNATIONAL SOCIETY FOR OPTICAL ENGINEERING 2001 SPIE US, vol. 4206, 5 November 2000 (2000-11-05), pages 64-71, XP002543915**
• **ALUPOAEI C E; OLIVARES J A; GARCIA-RUBIO L H: "Quantitative spectroscopy analysis of prokaryotic cells: vegetative cells and spores" BIOSENSORS & BIOELECTRONICS ELSEVIER UK, vol. 19, no. 8, 15 March 2004 (2004-03-15), pages 893-903, XP002543916 ISSN: 0956-5663**
• **CATALINA ELENA ALUPOAEI: "Modeling of the Transmission Spectra of Microorganisms", THESIS,, 1 December 2001 (2001-12-01), page 110PP, XP009128434,**

**Description**

This invention relates to the identification of microbial bodies, and more particularly to the quantitative interpretation of the multiwavelength spectra of microorganisms and cells.

BACKGROUND OF THE INVENTION

Multiwavelength Uv-vis spectra of microorganisms and cell suspensions contain quantitative information on their number, size, shape, chemical composition, and internal structure. These properties constitute essential information for the identification and classification of microorganisms and cells.

Characterization and classification of living organisms is a major objective in all branches of the biological sciences. There are many techniques used for the characterization of microorganisms such as microscopy, electron microscopy and ultraviolet microscopy, biomedical characteristics, chemical characterization and the like. Typical procedures include cell cultures which are time consuming and relatively expensive. For example, the specific information about the cell size and shape contained in the photomicrograph is quite limited and many cells have to be counted to obtain meaningful statistics.

Microorganisms have differences in chemical composition at the molecular level. At the macroscopic level they also have differences in size, shape and cell morphology. Different molecular cell compositions result in distinct spectroscopic patterns. Accordingly, spectroscopy techniques permit the classification and identification of microorganisms by their spectra.

Multiwavelength transmission spectra consist of combined absorption and scattering phenomena resulting from interaction of light with the microorganisms or cells typically suspended in a non-absorbing media. The distribution of intensities as a function of wavelength depends on the optical properties of the sample (see for example Y.D. Mattley et Al., Proceedings of the SPIE, vol. 4206, p.64-71, 2001). The complexity of microorganisms in terms of their chemical composition and internal structure makes the interpretation of their spectral signature a difficult task.

In the prior art, spectral signatures may be used to identify an unknown sample. However, the sample parameters of the unknown must substantially match those used to generate the known signature. In non-laboratory environments rapid identification of microorganisms cannot presently be achieved.

Accordingly, what is needed in the art is a model for the interpretation of the multiwavelength spectra of microorganisms. Such a model could be employed to interpret the spectra of microorganisms in non-laboratory settings. Because the model can be dynamically adjusted to match the conditions of the sample, the predicted spectra can be constructed and the sample swiftly identified.

It is, therefore, to the effective resolution of the aforementioned problems and shortcomings of the prior art that the present invention is directed.

However, in view of the prior art in at the time the present invention was made, it was not obvious to those of ordinary skill in the pertinent art how the identified needs could be fulfilled.

SUMMARY OF THE INVENTION

The invention is defined in claim 1.

The present invention comprises a model for the interpretation of the multiwavelength spectra of microorganisms. The model is based on light scattering theory, spectral deconvolution techniques, and on the approximation of the frequency dependent optical properties of the basic constituents of living organisms. The optical properties as functions of wavelength, and available literature data on size and chemical composition of *E. Coli* cells and *B. globigii* spores have been used to establish the sensitivity of the calculated spectra to the model parameters and to demonstrate that the model can reproduce the features of experimentally measured spectra. The model is used to deconvolute measured spectra in terms of critical parameters necessary for the detection and identification of cells, such as the size, dry mass, dipicolinic acid and the nucleotide concentration. The present invention has been showed to yield meaningful estimates of the size, dry mass, dipicolinic acid and the nucleotide concentration for *E. Coli, P. agglomerans, B. subtilis* spores, and the vegetative cells and spores of *B. globigii* from experimental data. Reliable estimates for cell size, number, chemical composition and indication of the characteristics of their internal structure and invention is applicable to a wide range of cell types found in diverse environments can be provided.

A method for detecting and identifying microorganisms from a multiwavelength spectra is provided including the steps of obtaining a specimen, resolving a sample spectrum for the specimen, calculating the average turbidity of the sample spectrum, normalizing the sample spectrum based on average turbidity, and comparing the sample spectrum to a library of known spectra to identify the specimen. The average turbidity is calculated by:

$$\bar{\tau} = \frac{1}{M} \sum_{i=1}^{M} \tau_{oi}$$

wherein M is the number of discrete data points taken, and $\tau_{oi}$ represents the turbidity measured at the $i^{th}$ wavelength. Preferably, the sample spectrum excludes wavelengths absorbed by a buffer solution in which the specimen is suspended.

In the event that the identity of the specimen cannot be satisfactorily determined by comparing the sample spectrum to the library of fingerprints a plurality of cellular component values from an optical property database for a known microorganism are selected. The plurality of component values may include those for cell chromophores, cellular structures, or at least one value of each. A model turbidity spectrum is calculated based on the sum of the cellular component values and the sum is compared with the sample spectrum to determine whether the sample spectrum is indicative of the known microorganism.

Previously, interpretation was limited to particle size and count in the range of 400 to 820 nm. The present invention now models cellular component values that contribute to the spectrum in wavelengths less than 400 nm such as nucleic acid (approximately at 260 nm) and dipicolinic acid (approximately at 275 nm). Accordingly, modeling of cellular components is substantially achieved through a 180 to 1100 nm spectrum range. As a plurality of component values may be employed in the model, the number of quantitative markers is increased, and thus, the accuracy and precision of the microorganism identification.

The model turbidity spectrum is calculated by:

$$\tau(\lambda_0) = N_p \ell \left(\frac{\pi}{4}\right) \sum_{i=1}^{M} x_i \int_0^\infty Q_{ext,i}(m_i(\lambda_0), D) D^2 f_i(D) dD$$

where D is the effective particle diameter, $Q_{ext}(m(\lambda_0), D)$ corresponds to the Mie extinction coefficient, $m(\lambda_0)$ is the complex refractive index, and $N_p$ is the number of particles per unit volume.

Preferably, the interpretation model divides the predetermined cell's structure into values for macrostructure, internal structure, and chromophores wherein the sum of the macrostructure, internal structure and chromophores values approximate the predetermined cell's turbidity spectrum. The equation for the total turbidity written in terms of the three distinct populations is:

$$\tau(\lambda_0) = N_p \ell \left(\frac{\pi}{4}\right) \begin{bmatrix} \left(x_1 \int_0^\infty Q_{ext,i}(m_i(\lambda_0), D) D^2 f_i(D) dD \right)_{\substack{Macro \\ Structure}} + \\ \left(x_2 \int_0^\infty Q_{ext,i}(m_i(\lambda_0), D) D^2 f_i(D) dD \right)_{\substack{Internal \\ Structure}} + \\ \left(x_3 \int_0^\infty Q_{ext,i}(m_i(\lambda_0), D) D^2 f_i(D) dD \right)_{\substack{Other \\ Structure}} \end{bmatrix}$$

Where $m_i$ (i =1-3) is the refractive index and $x_i$ (i =1-3) is the number fraction for each population,

$$\sum_{i=1}^{3} x_i = 1$$

The volume fraction of each population, $v_i$ within the microorganism is given by:

$$v_i = \frac{c_i}{c_{total}}$$

Assuming volume addivity, the total concentration, $c_{total}$ can be readily-calculated in terms of the concentration

of each population, $c_i$:

$$c_{total} = \sum_{i=1}^{N} c_i$$

Where N is the number of populations.

The real and imaginary parts of the complex refractive indices are functions of the chemical composition and can be calculated as a weighted sum of the contributions from the chromophores within each population:

$$n_i = \sum_{j=1}^{M} \omega_{ij} n_{ij}$$

$$k_i = \sum_{j=1}^{M} \omega_{ij} k_{ij}$$

Where $\omega_{ij}$ is mass fraction of the $j$th chromophore contained in the $i$th population, $n_i$ and $k_i$ real and imaginary refractive indexes of each population, and M is the total number of chromophoric groups. Adding the scattering contributions represented in Paragraph 00023 closes the total mass balance for each chromophoric group. Substituting the relations of Paragraphs 32-33 in the complex refractive index given by:

Substituting the relations of Paragraphs 32-33 in the complex refractive index, results the complex refractive index of the $i$th population:

$$m_i = \frac{\sum_{j=1}^{M} \omega_{ij} n_{ij}}{n_0} + \frac{i \sum_{j=1}^{M} \omega_{ij} k_{ij}}{n_0}$$

One application that may employ the present invention is that of a biosensor for detecting and identifying microorganisms from a multiwavelength spectra. The biosensor includes a spectrometer means adapted to measure the spectrum of a fluid sample. A processing means is communicatively coupled to the spectrometer means and a database is communicatively coupled to the processing means. A plurality of stored cellular component values representative of a microorganism model is stored in the database. The stored cellular component values may include scattering components such as the body of the cell, the cell wall, ribosomes, nucleic acid, cell inclusions and the iike. The stored cellular component values may also include chromophoric groups such as nucleic acids, amino acids, proteins, pigments, and toxins. It should be noted that chromophoric groups such as nucleic acids and dipicolinic acid, can be considered to be part of a particular scattering element (i.e., nuclei, chromosomes), or consider as scattering elements by themselves (i.e., plasmids). A plurality of stored cellular component values are selected for the model and their sum is calculated to predict the expected turbidity spectrum. A signal output means is provided whereby a value for the at least one quantifiable parameter of the fluid sample is measured by the spectrometer means and passed to the processing means, the processing means accesses the database for a stored quantifiable parameter in correlation with the at least one quantifiable parameter, responsive to a positive correlation, a signal is generated by the signal output means. A storage means communicatively coupled to the processing means monitors changes in quantifiable parameters in the fluid sample over time.

Accordingly, a first embodiment of the present invention is a method for detecting and identifying microorganisms from a multiwavelength spectra including the steps of obtaining a sample, acquiring the spectra from the sample, calculating the average turbidity of the sample spectra, normalizing the sample spectrum based on the average turbidity, and comparing the sample spectrum to a library of known spectra to identify the specimen. The average turbidity is calculated by:

4

$$\overline{\tau} = \frac{1}{M} \sum_{i=1}^{M} \tau_{oi}$$

wherein M is the number of discrete data point wavelengths taken, and $\tau_{oi}$ represents the turbidity measured at the $i^{th}$ wavelength and the sample spectrum excludes wavelengths absorbed by the suspending medium in which the sample is suspended. The multiwavelength spectra may utilize polarized light, non-polarized light, or a combination thereof.

It is therefore an object of the present invention to provide a method of identifying microorganisms that is reliable, efficient and fast.

It is another object of the present invention to provide a biosensor employing known spectroscopic technology capable of identifying microorganisms substantially without manual intervention.

It is to be understood that both the foregoing general description and the following detailed description are explanatory and are not restrictive of the invention as claimed. The accompanying drawings, which are incorporated in and constitute part of the specification, illustrate embodiments of the present invention and together with the general description, serve to explain principles of the present invention.

These and other important objects, advantages, and features of the invention will become clear as this description proceeds.

The invention accordingly comprises the features of construction, combination of elements, and arrangement of parts that will be exemplified in the description set forth hereinafter and the scope of the invention will be indicated in the claims.

BRIEF DESCRIPTION OF THE DRAWINGS

For a fuller understanding of the nature and objects of the invention, reference should be made to the following detailed description, taken in connection with the accompanying drawings, in which:

FIG. 1 is a diagrammatic of a prokaryotic cell.

FIG. 2 is a schematic representation of the absorption and scattering components for the quantitative interpretation model.

FIG. 3 is diagrammatic view of an embodiment of the invention utilizing normalization.

FIG. 4 is diagrammatic view of a comparative example utilizing deconvolution.

FIG. 5 is diagrammatic view of a comparative example utilizing deconvolution for both polarized and non-polarized light.

FIG. 6 is a diagrammatic view of a comparative example wherein calculation models are derived iteratively.

FIG. 7 is a diagrammatic view of a comparative example wherein predetermined models define the set of cellular elements selected from the optical property database.

FIG. 8 is a diagrammatic view of a comparative example wherein calculation models are derived from known microbiology of microorganisms.

FIG. 9 is a normalized optical density spectra from suspension of *E. Coli, P. agglomerans,* and the vegetative cells *of B. globigii.*

FIG. 10 is a comparison between the normalized optical density spectra from suspensions of spores and vegetative cells *of B. globigii.*

FIG. 11 is a comparison between the normalized optical density spectra from suspension of spores of *B. globigii* and *B. subtilis.*

FIG.12 is a comparison between measured and calculated optical density spectra of *E. coli.*

FIG. 13 is a comparison between measured and calculated optical density spectra of P. *agglomerans.*

FIG. 14 is a comparison between measured and calculated optical density spectra *of B. globigii* vegetative cells.

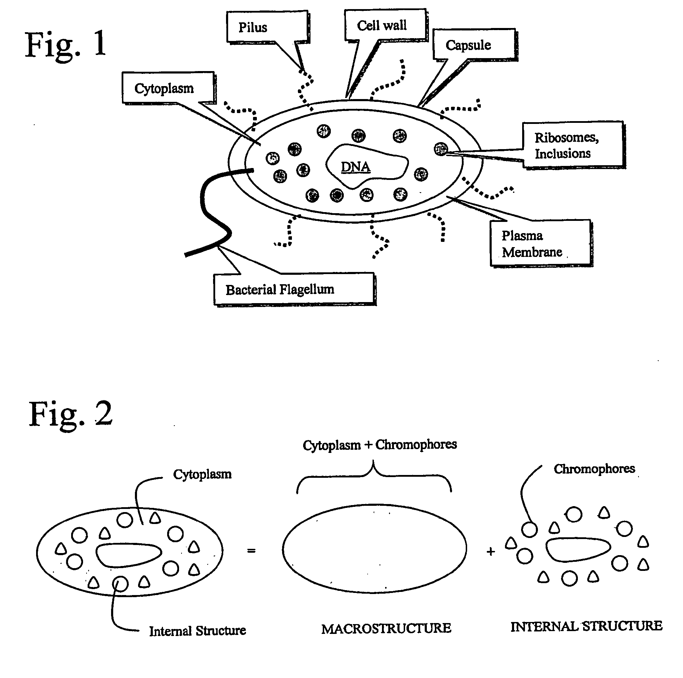

FIG. 1 is a comparison between measured and calculated optical density spectra of *B. globigii* spores.

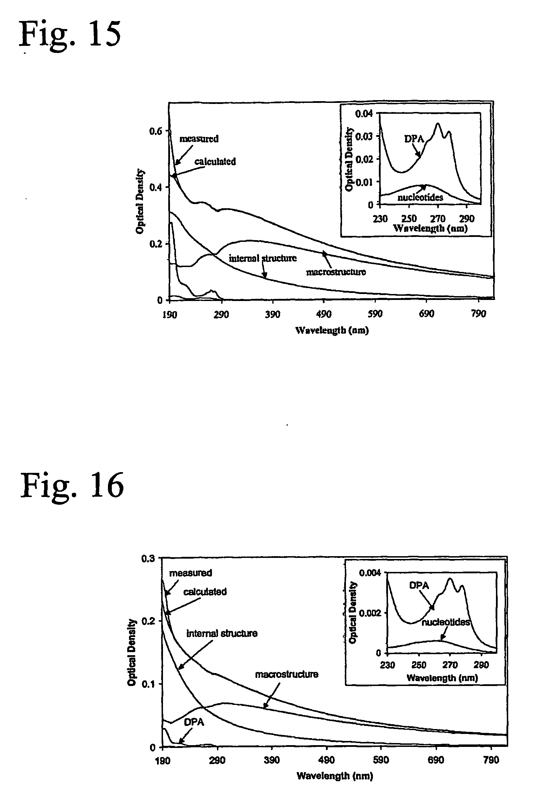

FIG. 16 is a comparison between measured and calculated optical density spectra of *B. subtilis* spores.

DETAILED DESCRIPTION OF THE PREFERRED EMBODIMENT

The present invention is based on Mie theory, where the volume of the microorganisms is expressed in terms of an equivalent sphere, and where the complex structure of microorganisms is approximated by dividing it into M groups or populations, each of which is characterized by its corresponding scattering and absorption components. The total scattering and absorption components of the spectrum will be given by the weighted sum of the contributions from the selected M structures or populations:

## EQUATION 1:

$$\tau(\lambda_0) = N_p \ell \left(\frac{\pi}{4}\right) \sum_{i=1}^{M} x_i \int_0^\infty Q_{ext,i}(m_i(\lambda_0), D) D^2 f_i(D) dD$$

Where $x_i$ (i = 1 → M) is the number fraction corresponding to each population such that,

## EQUATION 2

$$\sum_{i=1}^{M} x_i = 1$$

The complex refractive index $m_i(\lambda_0)$ is function of the chemical composition and can be calculated as a weighted sum of the contributions from the N Chromophores within each population:

## EQUATION 3

$$m_i(\lambda_0) = \frac{\sum_{j=1}^{n} \omega_{ij} n_{ij}}{n_0} + \frac{i \sum_{j=1}^{n} \omega_{ij} k_{ij}}{n_0}$$

Where $\omega_{ij}$ is mass fraction of the $j^{th}$ chromophore contained in the $i^{th}$ population, $n_i$ and $k_i$ correspond to the real and imaginary refractive indices of each population. Adding the scattering contributions represented by Equations 1 and 2 closes the total mass balance for each chromophoric group. The diameter in Equation 1 can be calculated from the closest geometrical approximation to the shape of the microorganisms. Assuming volume additivity, the total concentration can be readily calculated in term of the concentration of each population or structure.

Prokaryotic cells were used to validate the utility of the present invention. Prokaryotic cells are unicellular organisms with several architectural regions as shown in Fig. 1. appendages (proteins attached to the cell surface) in the form of flagella and pili (extensions of the membrane that allow bacteria to stick to substances, and to each other); a cell envelope consisting of a capsule (which protects the cell from the immune system of its host); cell wall and plasma membrane; and a cytoplasmic region that contains the cell genome (total DNA content), ribosomes, and various sorts of inclusions. The main scattering elements that can be readily identified are the body of the microorganism, the surrounding cell wall, the ribosomes, the nucleic acid structures, and the inclusions. The absorption components include nucleic acids, nucleotides, and the amino acids present in proteins. For some microorganisms, specific pigments and/or toxins may be present

A starting point for the modeling of the spectra of prokaryotic cells is to represent the complex structure of the microorganisms by dividing it into two main absorption and scattering groups or populations (M=2) containing a maximum of three groups of substances (N=3). The two populations are: (1) the body of the microorganism and its characteristic dimensions; and (2) an average representation of the internal structure through a characteristic dimension. The chemical composition of these structures will consist, as a first approximation, of nucleic acids, dipicolinic acid, and non-chromophoric substances such as proteins, lipids, and the like. Fig. 2 shows a schematic representation of the model dividing the macrostructure from the internal structure.

An additional simplification results from the low sensitivity shown by the simulated spectra to the variances of these two populations, and from the electron microscopy observations suggesting that the samples of vegetative cells and spores analyzed were narrowly distributed. Under these approximations, Equation 1 reduces to:

## EQUATION 4:

$$\tau(\lambda_0) = N_p \ell\left(\frac{\pi}{4}\right)\left(x_1 Q_{1ext}(m_i(\lambda_0), D_1)D_1^2 + (1-x_1)Q_{2ext}(m_2(\lambda_0), D_2)D_2^2\right)$$

Where the subscript 1 corresponds to the characteristics of the macrostructure, and 2 to corresponding elements of the internal structure. Equations 3 and 4 constitute the initial equations for the interpretation of the spectra.

*E. Coli* (JM 109ATCC #53323) and *B. globigii* spores (ATCC #9372) were obtained from the American Type Culture Collection (ATCC) Manassas, Virginia, *P. agglomerans* and *B. subtilis* were provided by Los Alamos Rational Laboratory, Los Alamos, New Mexico. Free carboxyl and amino groups (i.e., tyrosine) used for the representation of terminal amino acid groups and the n-acetyl/ethyl ester derivatives used to represent chromophores imbedded in protein molecules were purchased from Sigma Aldrich, Saint Louis Missouri. The dipicolinic acid was also from Sigma-Aldrich.

The Uv-vis transmission spectra from the cells suspensions were recorded using a diode array spectrometer (HP 8443 Hewlett-Packard, Palo Alto, CA) having an acceptance angle smaller than 2°. All measurements were conducted at room temperature using a 1 cm pathlength cuvette. Prior to running each sample, the spectrometer was zeroed to account for any stray light. To avoid the effect of inhomogeneities in the suspending medium, the background spectrum was taken using the corresponding suspending media from the batch utilized in the preparation of the original sample (sterilized de-ionized water). To eliminate concentration and particle number effects, the transmission spectra were normalized with the average optical density between 230-900 nm. To illustrate better the composition information, the first derivative of the spectra was numerically evaluated.

Because biologically pure cultures do not necessarily imply spectroscopically pure materials, special care is taken in washing and separating the cultures. This is particularly critical when the growth media has strong absorption characteristics. For these cases, prior to the spectroscopy measurements, the cells were washed in sterilized de-ionized water according to the following protocol; The microfuge tubes containing the sample were placed a Beckman Microfuge 12 and spun for four minutes. The tubes were removed from the microfuge and using a 1.0 ml pipette, the supernatant was slowly drawn off and discarded. A small amount of the fluid was left in each tube to avoid disturbing the pellets. The remaining pellets were resuspended in sterilized deionized water and vortexed for few seconds. The washing process was repeated at least two times. Spectroscopy measurements were taken to ensure the elimination of the growth media. The washing process was repeated until no changes in the spectra were observed. After the last washing, the pellet of clean cells was resuspended in sterilized deionized water, which was also used to dilute the samples. The level of dilution was selected to yield optical density values below 1.2 Absorbance Units (Au). When samples of pure cells (spores and vegetative cells) were available, they were directly suspended and diluted in sterilized de-ionized water prior to spectroscopy measurements.

The Mie scattering coefficients in Equation 4 were calculated with a computer program, which includes multiwavelength spectral calculations and has been adapted to calculate distributions of particle sizes. This program has been validated against published computer codes and tables. The refractive index of water $n_0(\lambda_0)$ in Equation 2 as a function of wavelength was calculated from the correlation reported by Thormählen.

The average refractive index pertaining to the scattering groups approximating the cell macrostructure were estimated using a two parameter Cauchy equation. The macrostructure parameters obtained for the microorganisms studied are given in Table I together with refractive index values reported in the literature.

TABLE I:

| | Parameter Values | | | | | Literature Values | |
|---|---|---|---|---|---|---|---|
| | E. Coli | P. agglome rans | B. globigii Veg. Cell | B. globigii spores | B. subtilis | Vegetative cells | Spores |
| $a_0$ | 1.3776 | 1.4538 | 1.3925 | 1.4993 | 1.4928 | - | - |
| $b_0$ | 3034.91 | 1984.92 | 2849.23 | 4393.90 | 4983.42 | - | - |
| $n_{542}$ | 1.3879 | 1.4605 | 1.4021 | 1.5142 | 1.5098 | 1.386-1.400 | 1.52-0.01 |
| % water | 77.54 | 47.59 | 71.63 | 24.92 | 26.75 | 72-78 | 18-26 |

With the exception of *P. agglomerans,* the estimated refractive indices are within the expected range. The reason for this discrepancy is not known at the present time, but it translates into lower water content than expected for vegetative cells (see last row in Table I).

TABLE II:

| | Parameter estimates | | | | |
|---|---|---|---|---|---|
| Microorganism | E. Coli | B. globigii veg. cells | B. globigii spores | P. agglomerans | B. subtilis spores |
| Volume, $\mu m^3$ | 1.26 | 1.230.782 | 0.43 | 0.43 | 0.59 |
| Equiv. diameter +/- 0.05 $\mu m$ | 1.34 | 1.33 | 1.14 | 0.93 | 1.04 |
| DNA+RNA content/cell +/- 10% | $7.70 \times 10^{-14}$ | $3.64 \times 10^{-14}$ | $1.52 \times 10^{-14}$ | $2.02 \times 10^{-14}$ | $16.84 \times 10^{-16}$ |
| DPA, g/cell +/- 5% | - | - | $4.86 \times 10^{-14}$ | - | $70.06 \times 10^{-16}$ |
| Fraction of internal structure, +/- 3% | 28.5 | 30.04 | 41.47 | 31.17 | 61.42 |
| Average size internal structure, +/- 5 nm | 80.76 | 72.84 | 132 | 66.98 | 128 |

The refractive index parameters for the internal structure were estimated under the assumption that primarily non-absorbing proteins compose the internal scattering elements. Analysis of the data reported in the literature resulted in the following estimates:

## EQUATION 5:

$$n(\lambda_o) = 1.55 + \frac{5900}{\lambda_o^2}$$

The optical properties corresponding to chromophoric groups incorporated to the scattering elements in Equations 3-4 were estimated. Whenever possible the measured data was compared with literature reports and found to be in good agreement.

Application of the interpretation model to the measured spectra results in quantitative differences between vegetative cells, vegetative cells and spores, and between different types of spores. For this purpose equations 4-5 have been implemented within a standard Marquardt-Levenberg Least-Squares algorithm. The parameters estimated include the average size of the microorganism (macrostructure), the average size of the internal scattering structures, the volume fraction of the internal structure, and the chemical composition in terms of the total nucleotide and dipicolinic acid concentrations. Although within the formulation of the interpretation model it is possible to consider the effect of the chromophores within each of the absorption and scattering structures (Equations 1-4), initially, only two hypothesis have been evaluated, namely whether the chromophoric groups absorb and scatter with the macrostructure or as part of the internal structure of the microorganisms. The parameter estimation and the evaluation of the two hypotheses were conducted conditional upon the values of the refractive index of the macrostructure for each microorganism (Table I), and the average values of the refractive index of the internal structure (Equation 5). The spectral data used for the estimation of the parameters was limited to the range 220-900 nm. This was done to minimize the effects of fluctuations in the composition of the suspending media.

There are many issues related to the spectroscopy of microorganisms and the identification of their characteristic fingerprints. The spectrometers and the measurement of the spectra do not represent a major technical challenge. However, the sampling process, the sample preparation, the state of growth of the microorganisms, and their natural variability in terms of size, shape and chemical composition can introduce considerable differences in their spectral response. For the identification of characteristic fingerprint of microorganisms, and for the development of interpretation models it is necessary to fix some of these variables. The reproducibility of the spectral measurements reported herein has been confined to cell suspensions prepared in sterilized de-ionized water.

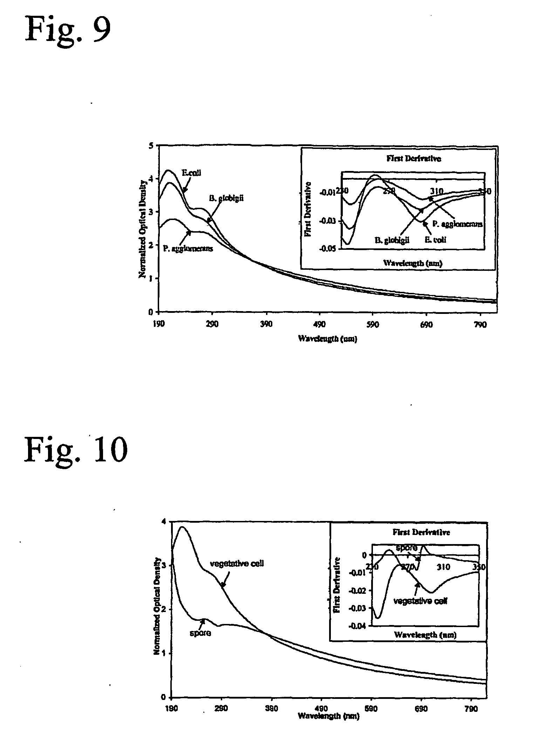

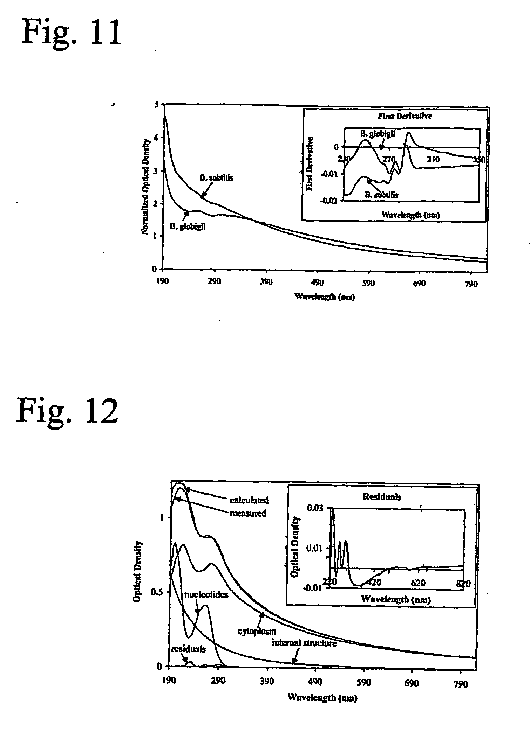

Previous reports have shown that the Uv-vis spectra of different types of microorganisms may vary considerably. In this report bacteria, vegetative cells and spores are analyzed to explore the potential of multiwavelength transmission measurements to discriminate between them. Fig. 9 shows the normalized optical density spectra and the first derivative spectra (inset) measured for *E. Coli, P. agglomerans,* and The vegetative cells of *B. globigii.* Notice that although the spectra appear to be somewhat similar, they are not identical; this is probably due to differences in chemical composition, in particular DNA and RNA. The spectral differences can be appreciated particularly in the first derivative spectra, which enhance the differences in chemical composition (inset Fig. 10). The similarity in the spectra is expected since these organisms have similar sizes and water content. Fig. 11 shows a comparison between the spores and vegetative cells *of B. globigii.* There is a marked difference between the spectra of the spores and the vegetative cells. These differences are expected since only the spores contain dipicolinic acid. Furthermore, the presence of high concentrations of calcium makes the spores highly refractive and as a result their scattering properties are affected. Comparison of the spectra from *B. globigii,* and *B. subtilis* spores show considerable differences (Fig. 12). The first derivative spectra shown in the inset has the same absorption bands, but clearly with varying intensity. The experimental measurements of the multiwavelength transmission spectra show evidence of quantifiable differences between the bacterial cells and the spores analyzed.

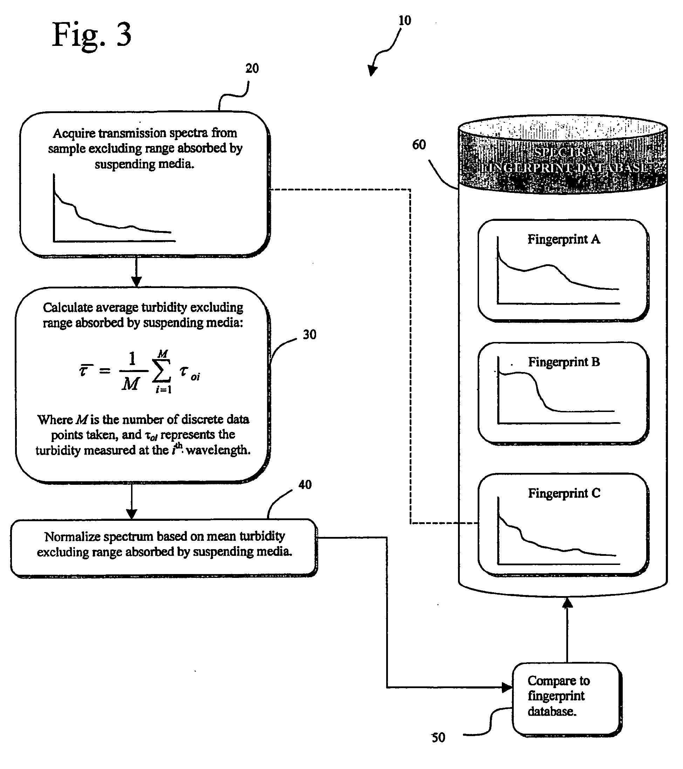

In Fig. 3, the transmission spectra from a sample is acquired 20. Preferably, the wavelengths absorbed by the suspending media are excluded. The average turbidity is calculated **30** by:

$$\overline{\tau} = \frac{1}{M} \sum_{i=1}^{M} \tau_{oi}$$

wherein M is the number of discrete data point wavelengths taken, and $\tau_{oi}$ represents the turbidity measured at the $i$th wavelength and the sample spectrum excludes wavelengths absorbed by the suspending medium in which the sample is suspended. The spectrum is normalized **40** based on the mean turbidity **30** excluding the range absorbed by the suspending media. A spectra fingerprint database **60** is accessed and the acquired spectra **20** is compared to known spectra fingerprints in the database **60** for a match.

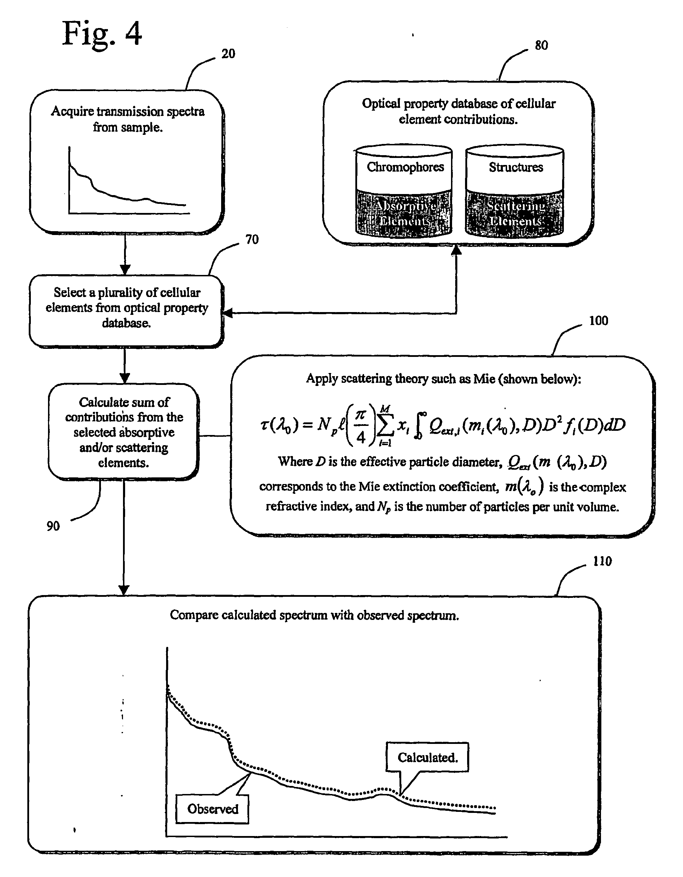

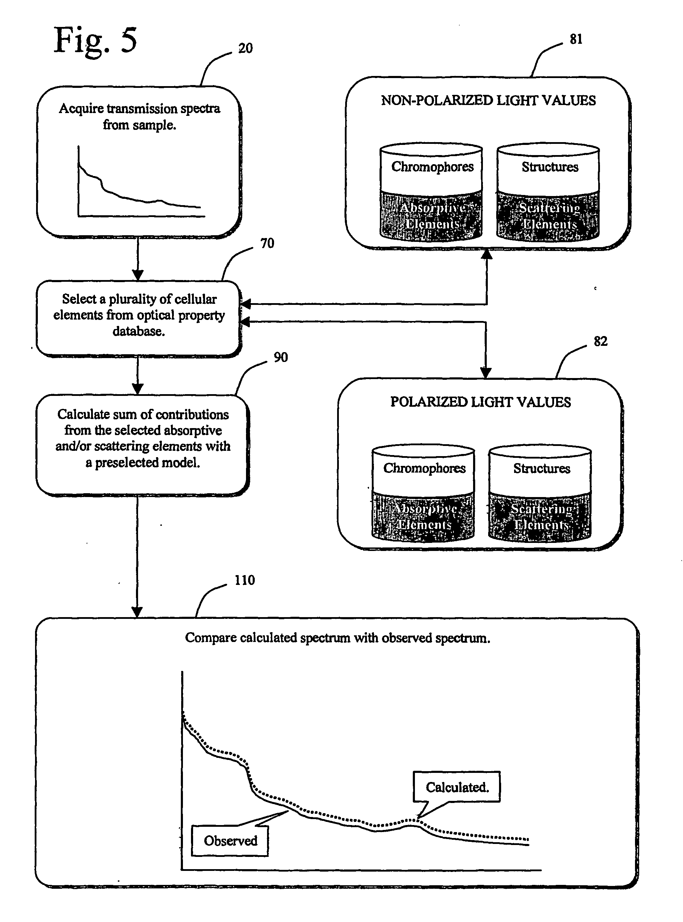

Fig. 4 illustrates a comparative example using deconvolution to identify the acquired transmission spectra **20** wherein a plurality of cellular elements are selected **70** from an optical property database **80** of cellular element contributions. The sum of the contributions is calculated **90** from the selected absorptive and/or scattering elements selected. An appropriate scattering theory **100** may be applied such as Mie as known by those skilled in the art. The calculated spectrum **90** and the observed spectrum **20** are then compared **110** to determine the presence of the microorganism in the sample. In Fig. 5, an alternative embodiment of Fig. 4 is presented wherein non-polarized **81** and polarized **82** values are provided to enable more precise deconvolution of the acquired spectra **20.**

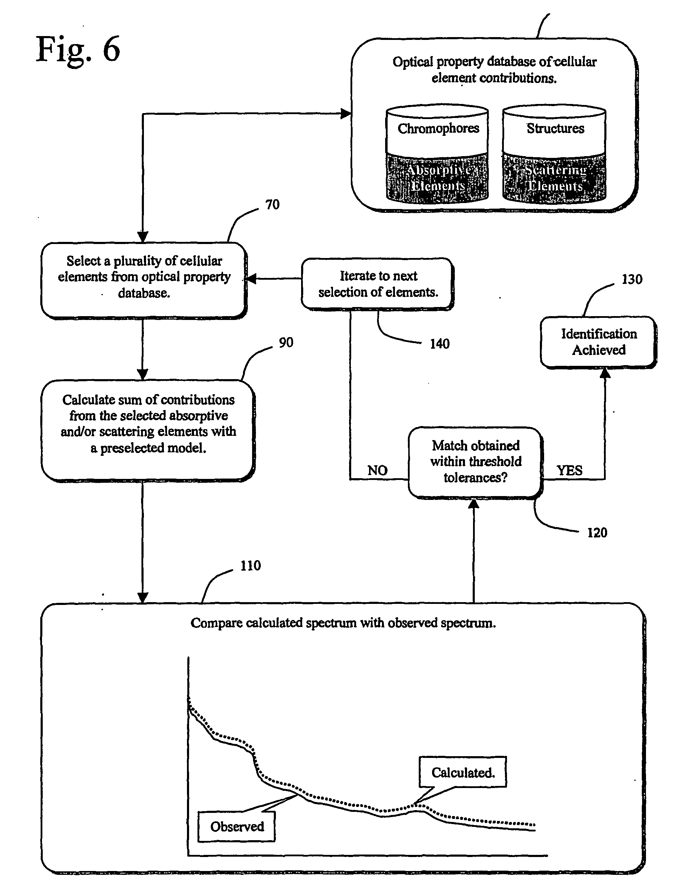

In Fig. 6, a plurality of cellular elements are selected **70** from the optical property database **80** and the sum of contributions are calculated **90**. The calculated and observed spectrums are compared **110** and evaluated **120** to determine whether a match is achieved under predetermined threshold tolerances. It is preferred that the spectrums be compared using a least-squares approach, but alternative methods known to those skilled in the art are acceptable. If an identification is achieved **130,** the microorganism associated with the preselected model is deemed to be present in the sample. Alternatively, if the match is not achieved within threshold tolerates, a new combination of selected elements may be executed **140** iteratively and the process repeated.

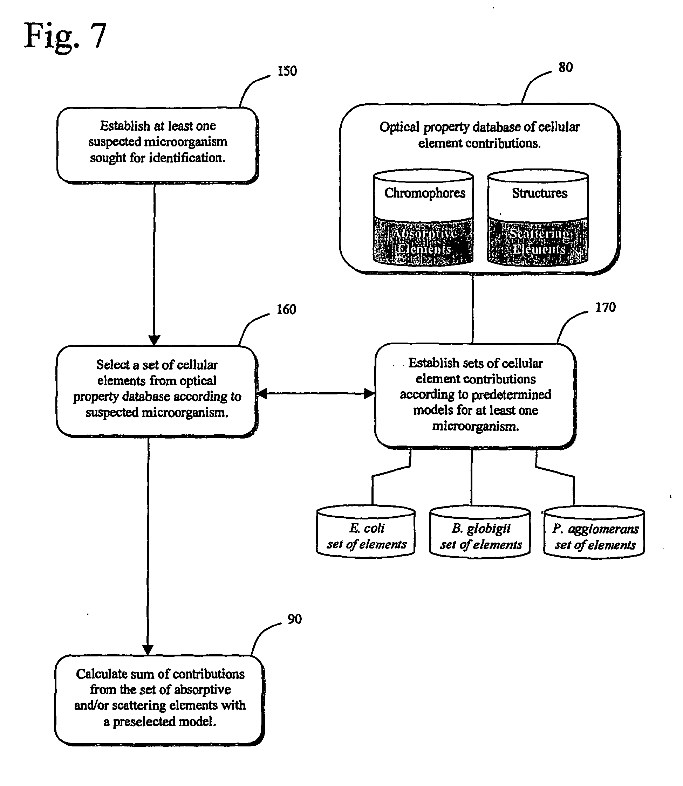

Fig. 7 illustrates the process of employing preexisting models for microorganisms in the present invention wherein at least one suspected microorganism is established **150** for identification. An array of established sets of cellular elements is accessed **170** containing predetermined models for at least one microorganism. The established sets **170** are derived from the optical property database **80.** A set of cellular elements is selected **160** which corresponds to the predetermined model for the suspected microorganism previously selected **150** and the sum of contributions is calculated **90** to determine if the suspected microorganism is present in a sample.

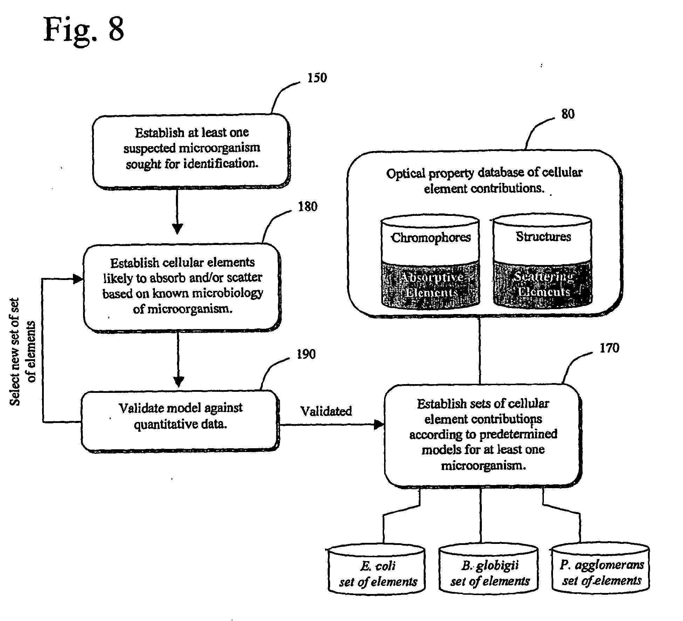

In Fig. 8, at least one suspected microorganism is established **150** for identification. The cellular elements likely to absorb and/or scatter are established **180** based on known microbiology for the at least one suspected microorganism. The combination of cellular elements selected are then validated **190** against quantitative data. Upon validation, the combination forms a model **170** for the at least one microorganism. If validation is not achieved, a new combination of cellular elements are selected and the validation process is repeated.

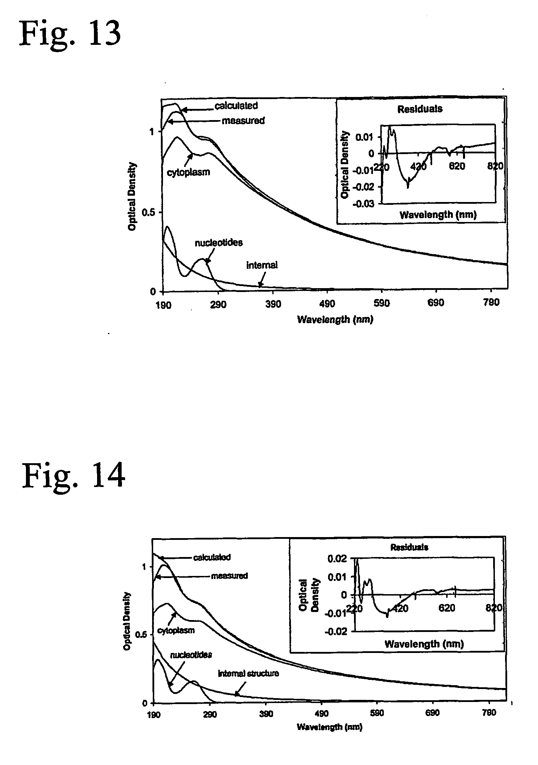

Figs. 13-16 show typical comparisons between measured and calculated spectra for three vegetative cells and for *B. globigii,* and *B. subtilis* spores. The contribution to the calculated total optical density from each of the model components, the total absorption of the corresponding chromophoric groups calculated as Beer-Lambert absorption, and the residuals are also included in the figures. Notice the spectral features contributed by each component and the dramatic effect of the chromophoric groups to the total optical density. Extinction coefficient is a spectroscopic term applied to a molecular group for determining absorption at a particular wavelength. The two structures and the chromophoric groups contribute differently over different portions of the spectra. Although the residuals show some correlation, it is evident that the calculated spectra adequately represents the measured spectra of a large variety of prokaryotic microorganisms.

The values for the parameters estimated for all the microorganisms studied are reported in Table II together with their approximate estimations errors. In addition to the parameter estimates, Table II also shows the literature data available for the volume of the microorganisms and the chromophore concentrations. The estimates of the particle volumes and concentrations are within the range of values reported in the literature that were obtained with completely different methods. The good agreement with literature values is surprising given the simplifying assumptions of the model. There are two outstanding values: the first one is the relatively small volume estimated for P. *agglomerans,* which could explain the low water content reported in Table I. The second value corresponds to the low DPA concentration estimate for *B. subtilis.* Notice however, that the DNA concentration estimates are in good agreement with the literature reports for this microorganism. The total nucleotide concentration estimated is in excellent agreement with the literature for *E. Coli.* Similarly, the DPA concentration for *B. globigii* obtained from the spectral deconvolution is in good agreement with the values reported in the literature for spores.

The correlation observed in the residuals can be explained on the basis of the approximations and simplifications used. In particular, a spherical equivalent scattering approximation has been used. Only the average sizes for each population of microorganisms and for the internal structure have been estimated. Also, an average representation of the total nucleotide absorption has been utilized, and the contribution of chromophoric amino acids has been neglected. The last two may account for the correlation in the residuals apparent in the region between 230-300 nm. One may also include hypochromic effects, which are known to alter the spectral features of strong chromophoric groups such as DNA and RNA. This model is capable of representing the spectroscopy behavior of complex biological structures such as vegetative cells and spores, and as such, it provides quantitative information for analysis and differentiation.

It will be seen that the objects set forth above, and those made apparent from the foregoing description, are efficiently attained and since certain changes may be made in the above construction without departing from the scope of the invention, it is intended that all matters contained in the foregoing description or shown in the accompanying drawings shall be interpreted as illustrative and not in a limiting sense.

It is also to be understood that the following claims are intended to cover all of the generic and specific features of the invention herein described, and all statements of the scope of the invention which, as a matter of language, might be said to fall therebetween. As microorganisms pervade nearly every environment on Earth, affect the health of billions of humans and animals daily, are critical to numerous commercial industries, and are of concern as potential military and terrorist weapons, the scope of applications for the present invention should not be limited by exemplary uses described herein. Now that the invention has been described,

## Claims

1. A method for detecting and identifying microorganisms from a multiwavelength spectra comprising the steps of:

    obtaining a sample;
    acquiring the spectra from the sample;
    calculating the average turbidity of the sample spectra;
    normalizing the sample spectrum based on the average turbidity; and

comparing the sample spectrum to a library of known spectra to identify the specimen.

2. The method of claim 1 wherein the average turbidity is calculated by $\bar{\tau} = \frac{1}{M} \sum_{i=1}^{M} \tau_{oi}$ wherein $M$ is the number of discrete data point wavelengths taken, and $\tau_{io}$ represents the turbidity measured at the $i$th wavelength.

3. The method of claim 1 wherein the sample spectrum excludes wavelengths absorbed by the suspending medium in which the sample is suspended.

4. The method of claim 1 wherein the multiwavelength spectra comprises polarized light.

5. The method of claim 1 wherein the multiwavelength spectra comprises non-polarized light.

6. The method of claim 1 wherein the multiwavelength spectra comprises polarized light and non-polarized light.

**Patentansprüche**

1. Verfahren zum Nachweisen und Identifizieren von Mikroorganismen aus einem Multiwellenlängenspektrum, das folgende Schritte umfasst:

   Gewinnen einer Probe;
   Aufnehmen des Spektrums von der Probe;
   Berechnen der durchschnittlichen Trübung des Probenspektrums;
   Normalisieren des Probenspektrums anhand der durchschnittlichen Trübung und
   Vergleichen des Probenspektrums mit einer Bibliothek bekannter Spektren zum Identifizieren der Probe.

2. Verfahren nach Anspruch 1, wobei die durchschnittliche Trübung mit $\bar{\tau} = \frac{1}{M} \sum_{i=1}^{M} \tau_{oi}$ berechnet wird, wobei

$M$ die Anzahl der erfassten diskreten Datenpunktwellenlängen ist und $\tau_{oi}$ die Trübung ist, die bei der i-ten Wellenlänge gemessen wird.

3. Verfahren nach Anspruch 1, wobei das Probenspektrum Wellenlängen ausschließt, die vom Suspensionsmedium, in dem die Probe suspendiert ist, absorbiert werden.

4. Verfahren nach Anspruch 1, wobei das Multiwellenlängenspektrum polarisiertes Licht umfasst.

5. Verfahren nach Anspruch 1, wobei das Multiwellenlängenspektrum nicht polarisiertes Licht umfasst.

6. Verfahren nach Anspruch 1, wobei das Multiwellenlängenspektrum polarisiertes Licht und nicht polarisiertes Licht umfasst.

**Revendications**

1. Procédé de détection et d'identification de microorganismes à partir de spectres à multiples longueurs d'ondes comprenant les étapes de :

   obtention d'un échantillon ;
   acquisition des spectres à partir de l'échantillon ;
   calcul de la turbidité moyenne des spectres de l'échantillon ;
   normalisation du spectre de l'échantillon sur la base de la turbidité moyenne ; et
   comparaison du spectre de l'échantillon avec une banque de spectres connus afin d'identifier l'échantillon.

2. Procédé selon la revendication 1, dans lequel la turbidité moyenne est calculée par $\bar{\tau} = \frac{1}{M} \sum_{i=1}^{M} \tau_{oi}$ dans

lequel M est le nombre de point de données de longueurs d'ondes discrets acquis et $\tau_{oi}$ représente la turbidité mesurée à la $i^{ème}$ longueur d'onde.

3. Procédé selon la revendication 1, dans lequel le spectre de l'échantillon exclut des longueurs d'ondes absorbées par le milieu de suspension dans lequel l'échantillon est mis en suspension.

4. Procédé selon la revendication 1, dans lequel les spectres à multiples longueurs d'ondes comprennent de la lumière polarisée.

5. Procédé selon la revendication 1, dans lequel les spectres à multiples longueurs d'ondes comprennent de la lumière non polarisée.

6. Procédé selon la revendication 1, dans lequel les spectres à multiples longueurs d'ondes comprennent de la lumière polarisée et de la lumière non polarisée.

# Fig. 1

Pilus

Cell wall

Capsule

Cytoplasm

Ribosomes, Inclusions

DNA

Plasma Membrane

Bacterial Flagellum

# Fig. 2

Cytoplasm + Chromophores

Cytoplasm

Chromophores

Internal Structure

MACROSTRUCTURE

INTERNAL STRUCTURE

# Fig. 3

**20**

Acquire transmission spectra from sample excluding range absorbed by suspending media.

**60**

SPECTRA FINGERPRINT DATABASE

Fingerprint A

Fingerprint B

Fingerprint C

**30**

Calculate average turbidity excluding range absorbed by suspending media:

$$\bar{\tau} = \frac{1}{M} \sum_{i=1}^{M} \tau_{oi}$$

Where $M$ is the number of discrete data points taken, and $\tau_{oi}$ represents the turbidity measured at the $i^{th}$ wavelength.

**40**

Normalize spectrum based on mean turbidity excluding range absorbed by suspending media.

**10**

Compare to fingerprint database.

**50**

# Fig. 4

Acquire transmission spectra from sample. — 20

Optical property database of cellular element contributions. — 80

Chromophores | Structures

Absorptive Elements | Scattering Elements

Select a plurality of cellular elements from optical property database. — 70

Calculate sum of contributions from the selected absorptive and/or scattering elements. — 90

Apply scattering theory such as Mie (shown below): — 100

$$\tau(\lambda_0) = N_p \ell \left(\frac{\pi}{4}\right) \sum_{i=1}^{M} x_i \int_{0}^{\infty} Q_{ext,i}(m_i(\lambda_0), D) D^2 f_i(D) dD$$

Where $D$ is the effective particle diameter, $Q_{ext}(m(\lambda_0), D)$ corresponds to the Mie extinction coefficient, $m(\lambda_o)$ is the complex refractive index, and $N_p$ is the number of particles per unit volume.

Compare calculated spectrum with observed spectrum. — 110

Calculated.

Observed

# Fig. 5

**20** Acquire transmission spectra from sample.

**81** NON-POLARIZED LIGHT VALUES

Chromophores — Absorptive Elements

Structures — Scattering Elements

**70** Select a plurality of cellular elements from optical property database.

**82** POLARIZED LIGHT VALUES

Chromophores — Absorptive Elements

Structures — Scattering Elements

**90** Calculate sum of contributions from the selected absorptive and/or scattering elements with a preselected model.

**110** Compare calculated spectrum with observed spectrum.

Calculated.

Observed

16

# Fig. 6

Optical property database of cellular element contributions.

Chromophores

Absorptive Elements

Structures

Scattering Elements

70

Select a plurality of cellular elements from optical property database.

Iterate to next selection of elements.

140

130

Identification Achieved

90

Calculate sum of contributions from the selected absorptive and/or scattering elements with a preselected model.

NO

Match obtained within threshold tolerances?

YES

120

110

Compare calculated spectrum with observed spectrum.

Calculated.

Observed

# Fig. 7

```
                    150                                          80
┌──────────────────────────┐      ┌──────────────────────────────────┐
│   Establish at least one │      │  Optical property database of      │
│  suspected microorganism │      │       cellular element             │
│  sought for identification.│     │       contributions.               │
└──────────────────────────┘      │  ┌──────────┐    ┌──────────┐      │
            │                      │  │Chromophores│   │ Structures │     │
            │                      │  │ Absorptive │   │ Scattering │     │
            │                      │  │  Elements  │   │  Elements  │     │
            │                      │  └──────────┘    └──────────┘      │
            │                      └──────────────────────────────────┘
            │                 160                             170
            ▼                                     │
┌──────────────────────────┐      ┌──────────────────────────────────┐
│  Select a set of cellular │ ◄──► │   Establish sets of cellular       │
│  elements from optical    │      │   element contributions            │
│  property database        │      │   according to predetermined       │
│  according to suspected   │      │   models for at least one          │
│  microorganism.           │      │   microorganism.                   │
└──────────────────────────┘      └──────────────────────────────────┘
            │                              │         │         │
            │                        ┌─────────┐┌─────────┐┌─────────┐
            │                        │  E. coli ││B. globigii││P. agglomerans│
            │                        │ set of   ││ set of   ││ set of   │
            │                        │ elements ││ elements ││ elements │
            │                        └─────────┘└─────────┘└─────────┘
            │          90
            ▼
┌──────────────────────────┐
│  Calculate sum of         │
│  contributions from the   │
│  set of absorptive        │
│  and/or scattering        │
│  elements with            │
│  a preselected model.     │
└──────────────────────────┘
```

# Fig. 8

150

Establish at least one
suspected microorganism
sought for identification.

80

Optical property database of cellular
element contributions.

Chromophores

Structures

Absorptive
Elements

Scattering
Elements

180

Establish cellular elements
likely to absorb and/or scatter
based on known microbiology
of microorganism.

Select new set of set
of elements

190

Validate model against
quantitative data.

Validated

170

Establish sets of cellular
element contributions
according to predetermined
models for at least one
microorganism.

E. coli
set of elements

B. globigii
set of elements

P. agglomerans
set of elements

# Fig. 9

# Fig. 10

# Fig. 11

# Fig. 12

# Fig. 13

# Fig. 14

# Fig. 15

# Fig. 16

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

• **Y.D. Mattley et al.** *Proceedings of the SPIE,* 2001, vol. 4206, 64-71